# EUROPEAN PATENT APPLICATION

(11) **EP 1 508 556 A1**
(43) Date of publication of application: **23.02.2005**
(21) Application number: 04254768.7
(22) Date of filing: 07.08.2004
(51) Int. Cl.: C07C 2/70, C07C 15/085, C07D 333/08

(54) **Aromatic alkylation process in the presence of a, sulfonated, halogenated and base treated styrene DVB copolymer catalyst.**

(30) Priority: 20.08.2003 US 496473 P
(71) Applicant: Rohm and Haas Company, Philadelphia, Pennsylvania 19106-2399 (US)
(72) Inventor: Ramprasad, Dorai, Allentown Pennsylvania 18104 (US); Collin, Jennifer Reichl, Devon Pennsylvania 19333 (US)
(74) Representative: Kent, Venetia Katherine

(57) **Abstract**

The present invention provides a system, method and catalyst for catalyzing alkylation reactions. The method includes catalyzing an alkylation reaction using a base treated, sulfonated, halogenated, and optionally acid regenerated thermally stable catalyst.

## Description

This invention relates to a method of, system for, and catalyst for use in alkylation reactions. The invention also provides for a method of making the catalyst capable of alkylation, and a catalyst composition.

### BACKGROUND OF INVENTION

The alkylation of aromatic hydrocarbons with olefins is applied on a large scale in the chemical industry. Important reactions are the alkylation of benzene with propylene to give cumene, alkylation of benzene with ethylene to get ethylbenzene and the alkylation of toluene with propylene to get cymenes. These and other reactions use an aromatic hydrocarbon, i.e. a ring or fused ring system containing 4n+2 Pi electrons. Examples of these aromatic hydrocarbons include benzene, naphthalene or phenanthrene or even heterocyclic compounds such as pyrrole, thiophene or furan or pyridine. Cumene is used to make phenol and acetone. These compounds end up in important end products such as bis-phenol A which is used for polycarbonate based compact discs and other products. Phenol can be further alkylated with octene, nonene or dodecene to make products which are used as lubricant additives, antioxidants, surfactants, fuel additives, herbicides, insecticides, and fragrances.

Similarly, ethyl benzene is used to make styrene and cymene is used to make cresols. The alkylation of benzene with C₁₀-C₁₄ olefins are used to make linear alkylbenzenes ("LAB"). LAB's are used to make a surfactant detergent intermediate. The alkylation of thiophenes with olefins is used to reduce sulfur content in gasoline as described in U.S. Patent Nos. 5,599,441, and 5,863,419). However, one problem is that, in many of these industrial processes, the catalysts used are strong mineral acids or Lewis acids which are toxic and corrosive. There exists a need to solve this problem. Part of the solution to this problem includes replacing AlCl₃, HF and H₃PO₄ catalysts with more benign solid acid thermally stable catalysts. These conventional polymeric catalysts also suffer from the additional problems of thermal breakdown when used at high temperatures, and a lack of selectivity for desired end products. This problem can be solved by using methods and systems capable of alkylation at high temperatures and which produce a desired alkylation product with high selectivity.

The use of sulfonated polystyrene resins crosslinked with divinyl benzene as catalysts for aromatic (toluene and phenol) alkylation has been generally described in U.S. Patent No. 3,037,052, and British Patent No. 1,393,594. British Patent Nos. 1,393,594 and 956,357 describe a styrene divinyl benzene resin which is core halogenated. A problem with the processes and catalysts used in these patents involves chlorinated polymers that can split off HCl in addition to sulfuric acid during use. This problem leads to significant corrosion of stainless steel reactosr, and/or halide contamination of product. Similarly, U.S. Patent No. 5,863,419 describes the alkylation of thiophene at 200°C using Amberlyst™35 resin catalyst which is unstable at this temperature, and does not provide the requisite level of performance desired.

There exists a significant need in the art for methods of alkylation, and catalysts that can be used for alkylation that are thermally stable and provide improved performance characteristics including high selectivity for alkylation product, no or little degredation when used at high temperatures, and little or no reactor corrosion. In addition to solving other problems in the art, the present invention provides solutions to the problems mentioned above and satisfies the needs of the art.

The present invention provides a method of aromatic alkylation that includes adding a base treated, sulfonated, halogenated thermally stable catalyst to reactants. The catalyst comprises a styrene DVB copolymer, a styrene DVB/EVB copolymer, or a combination thereof. In one variant, the catalyst is surface sulfonated and acid regenerated.

In another aspect, a system for alkylation utilizes the method described herein, and a manufacturing facility utilizes the system described herein.

In yet another variant, the invention provides an alkylation product manufactured utilizing the method, system and manufacturing facility described herein, and downstream products made from the alkylation products.

These and other aspects of the invention and advantages other than those described above are also described in the detailed description, and examples herein.

A series of resins that are chlorinated with minimal leaching of HCl under reaction conditions and active alkylation catalysts were prepared. It was found that these resins concurrently provide higher activity than conventional catalysts in the art, while simultaneously providing low leaching levels of chlorine into reactor systems. One advantage of these resins is longer reactor life since the corrosion effects of the leached HCl are greatly minimized versus currently available resins. Moreover, a series of alkylation reactions can be run without interruption over longer periods of time since the replacement of components of alkylation reactors and systems is greatly reduced.

In one aspect, the present invention relates to a method of aromatic alkylation using a catalyst that includes a styrene divinylbenzune (DVB) copolymer, and/or a DVB and ethylvinylbenzene (EVB) copolymer. The method includes preparing an alkylation product by a method using a base treated, sulfonated or surface sulfonated, halogenated and acid regenerated thermally stable catalyst. Alkylation products can be prepared by the following methods: a) the reaction of an aromatic compound with an olefin, b) reaction of an aromatic compound and an alcohol, and c) reaction of an aromatic compound and an alkyl halide or alkyl ester.

In yet a further aspect, the invention provides an improved styrene DVB resin catalyst comprising 4-chlorostyrene aromatic groups and a polymer backbone. At least one of the aromatic groups has a chlorine in a styrenic para-position, and optionally other chlorines thereon. The polymer backbone is substantially free of leachable chlorine, and as a result provides the additional benefit of an increased reaction time, and a product in which the risk of halide contamination is greatly reduced or eliminated. The catalyst includes a halogenated DVB moiety. In one variant, the aromatic rings are oleum sulfonated, and as a result become polysulfonated. In yet another variant, the styrene DVB resin further comprises sulfone bridges. Another variant includes a 4-halo (F,Br, and/or I) styrene. Use of the catalysts, methods and systems described herein provides unexpected performance improvements in alkylation which are described below.

In another aspect, the invention provides a method of making a thermally stable catalyst that is used to catalyze alkylation reactions. The method includes base treating a sulfonated and halogenated copolymer to obtain a base treated copolymer, and regenerating the base treated copolymer with an acid. In another variant, the method of making a thermally stable catalyst for alkylation includes base treating a halogenated copolymer to obtain a base treated copolymer, and sulfonating the base treated copolymer.

In yet a further variant, the method of making a thermally stable catalyst for alkylation includes base treating a sulfonated and halogenated copolymer to obtain a base treated copolymer, and regenerating the base treated copolymer with an acid. The method of making a thermally stable catalyst for alkylation optionally includes chlorinating a styrene DVB copolymer, base treating the halogenated copolymer, and sulfonating the halogenated and base treated catalyst. The styrene DVB copolymer is a polysulfonated copolymer in another variant of the invention.

In yet a further aspect, the invention provides an improved styrene DVB resin catalyst comprising aromatic groups having more than one SO₃H moiety and a polymer backbone. The improvement includes halogenated aromatic groups, while concurrently providing a polymer backbone that is substantially free of leachable chlorine (or other detrimental halogens). The catalyst is used to catalyze alkylation reactions. In one variant, the amount of leachable chlorine is at least an order of magnitude less than the amount of leachable chlorine of a catalyst that is not base treated. In yet a further variant, the polymer backbone of the catalyst is free of leachable chlorine or other detrimental halogens.

Variants of the invention relate to preparation of resins of high thermal stability with low leaching of chlorine which are used as catalysts with high activity for catalyzing alkylation reactions. Several types of catalytic resins were prepared according to the reaction schemes shown below. The first reaction scheme for making a catalyst of the present invention involves the following process: Styrene DVB copolymer --→ Sulfonate or surface sulfonate -→ chlorinate (or halogenate) -→ base treat --→ regenerate with acid.

The chlorination (or halogenation) step incorporates chlorine (or other halogen) in the aromatic ring as well as the aliphatic backbone of the polymer. It is the chlorine (or other halogen) on the backbone that can undesireably leach of slowly as HCl (or other corrosive compound) in an alkylation process. Where this happens, accelerated equipment corrosion occurs resulting in undesirable down time and equipment replacement costs. Heating the polymer in a basic solution results in, for example, the leachable chlorine (or other detrimental halogen) being removed. Moreover, this process can be carried out in the space of a several hours in contrast to the art process which takes 10 days to carry out, e.g. U.S. Patent No. 4,705,808. It is appreciated that the time needed to manufacture a catalyst of the invention is greatly reduced to the range of several hours, e.g. 1-10 hours in one variant of the invention.

Using this process, a polymer is created that has a higher acid site density than that created by prior conventional methodologies. Consequently, a catalyst created by the process used herein has the advantage of lower desulfonation rates than catalysts produced by conventional processes. This results in higher catalytic activity since more active sites remain on the catalyst than after preparation using conventional processes.

Scheme 1 was used to chlorinate Amberlyst™ 39 resin catalyst, a commercially available sulfonated styrene DVB (7%) co-polymer from Rohm & Haas Company, Philadelphia, PA. It is appreciated that other resins could also be used herein having comparable DVB levels. After refluxing with a 2N sodium hydroxide solution for 22 hours, followed by regeneration with hydrochloric acid, a new resin(A) was obtained. The hydrolytic stability of this resin was tested at 200°C for 24 hours. Table 1 shows that Resin A loses 9.5% of its sulfonic acid groups versus 33.4% lost by resin **F** which is a conventional chlorinated resin containing 12% DVB. Additionally, the percentage chlorine detected in solution is much less for resin **A**. Resin **F** was also subjected to a base treatment to get resin **B** which has improved stability and lower chlorine leaching than resin **F**. Resin **F** was also post treated using the process described in U.S. Patent No. 4,705,808 to get resin C. It is clear from Table 1 that resin B has a higher acid capacity and is different from resin C in its properties and performance characteristics. Finally, scheme 1 was used to prepare resin G which is surface sulfonated and chlorinated, base treated and acid regenerated. A variant of the invention of scheme 1 involves a chlorination step followed by base treatment prior to the sulfonation. Additionally, a base treatment can be performed in a variety of solvents or mixtures of solvents. The optimum conditions are resin specific, and can readily be determined empirically. In the examples described, an aqueous 50% NaOH solution for 4 hrs at 130°C was used.

Scheme 2 is also used to obtain a catalyst of the present invention which is used to catalyze alkylation reactions: Styrene DVB copolymer --→ polysulfonate using oleum -→ chlorinate (or otherwise halogenate)-→ base treat --→ regenerate with acid. Oleum sulfonation of a styrene DVB co-polymer results in a polysulfonated polymer (one that has greater than one SO₃H group per ring). It is appreciated that the resin created by the process is both a polysulfonated and a chlorinated resin. Amberlyst™36 resin catalyst is a polysulfonated resin with 12% DVB. This resin was chlorinated and base treated as in Scheme 2 to get resin D. The thermal stability results of resin D (see Table 1) shows that it has the following performance characteristics: a high acid capacity, good stability and low chlorine leaching.

Scheme 3 is also used to obtain a catalyst of the present invention: 4-chlorostyrene (or other 4-halosytrene) DVB copolymer -→ sulfonate using oleum - → chlorinate (or otherwise further halogenate)-→ base treatment -→ regenerate with acid. A monomer is prepared using the technique of British Patent 1,393,594. A halogenated monomer such as chlorostyrene with a cross-linker such as divinylbenzene is used, thereafter one sulfonates the polymer using chlorosulfonic acid. This original polymer is unstable at high temperatures because the DVB part is not deactivated by a halogen atom. In this variant, the polymer is chlorinated to create desirable catalytic properties as well as solving the temperature stability problem by providing a catalyst having high temperature stability. Chlorination (or other halogenation) of the DVB creates a very stable resin since all the SO₃H groups on the chlorinated resin will generally be in a meta position.

In another variant of the invention, a copolymer of 4-chlorostyrene with 12% DVB was prepared and then sulfonated using oleum as the sulfonating agent for a short reaction time. This is a substantial improvement on the procedure described in British Patent 1,393,594. The resulting resin has sulfone bridges which increase thermal stability. The resin was then further chlorinated and then base treated to remove leachable chlorine and after acid regeneration yielded resin **E**. The thermal stability of this resin and the low chlorine leaching coupled with the high acid capacity provides the resin with important catalytic properties and unexpected performance characteristics. Scheme 3 can therefore be used to prepare a variety of styrene DVB polymers that are fully halogenated and have SO₃H groups in a meta position. This method can be also used to prepare polysulfonated chlorinated resins as in Scheme 2 by varying the sulfonation conditions. Additionally, the chlorination of the copolymer and base treatment can be performed prior to the sulfonation step.

It is further appreciated that the steps described in the various schemes mentioned above can be combined in various permutations such that the desired catalytic properties of the catalyst are obtained herein.

**TABLE 1**

| RESIN | %Cl | %S | MmolSO3H/ g | Mmol SO3H/g after 200C, 24hours | % loss in acid sites | % chlorine in liquid after 200C, 24 hours |
|---|---|---|---|---|---|---|
| **A** | 26.78, | 8.62 | 2.75 | 2.49 | 9.45 | 0.002 |
| **B** | 19.62, | | 3.31 | 2.50 | 24.60 | 0.19 |
| | 10.24 | | | | | |
| **F** | 22.00 | | 3.14 | 2.10 | 33.40 | 1.34 |
| **C** | 19.30 | | 3.06 | 2.12 | 30.40 | 0.08 |
| **D** | 10.04, | | 4.78 | 3.79 | 20.70 | 0.11 |
| | 16.69 | | | | | |
| **E** | 15.96, | | 4.16 | 3.52 | 15.34 | 0.12 |
| | 13.95 | | | | | |
| **G** | | | 0.55 | 0.29 | 46.4 | 0.004 |

### COMPRESSIBILITY OF RESINS

Chlorination (or other halogenation) of a monosulfonated styrene DVB resin reduces its compressibility. The change in the compressibility depends on the percent DVB in resin and on the degree of chlorination or other halogenation. The results are shown below.

| RESIN | COMPRESSIBILITY cm/m |
|---|---|
| Styrene 12% DVB copolymer monosulfonated | 4.03 |
| Styrene 12% DVB copolymer chlorinated to 20% by weight | 3.03 |
| Styrene 7% DVB copolymer monosulfonated | 7.23 |
| Styrene 7% DVB copolymer monosulfonated and chlorinated to 27% by weight | 4.39 |

### CATALYTIC ACTIVITY OF RESINS

The catalysts of this invention were tested for the reaction of benzene with propylene to produce cumene. A mixture of N₂/benzene/propylene in a 6.0/3.5/0.5 ratio was passed over 1g of heated catalyst at 150psig using a gas hourly space velocity (GHSV) of 12000 l/kg/h. The results in Table 2 were unexpected. C3 below is propene.

| Catalyst | C3 % conversion 120°C | C3 % conversion 130°C | Cumene % selectivity 120°C | Cumene % selectivity 130°C |
|---|---|---|---|---|
| **C** | 19.3 | | 99 | |
| | 10.6(second day) | 14.5 | 100 | 100 |
| **B** | 69.4 | 72.8 | 97.8 | 95.7 |
| **G** | 23.3 | 29.9 | 99.1 | 98.8 |

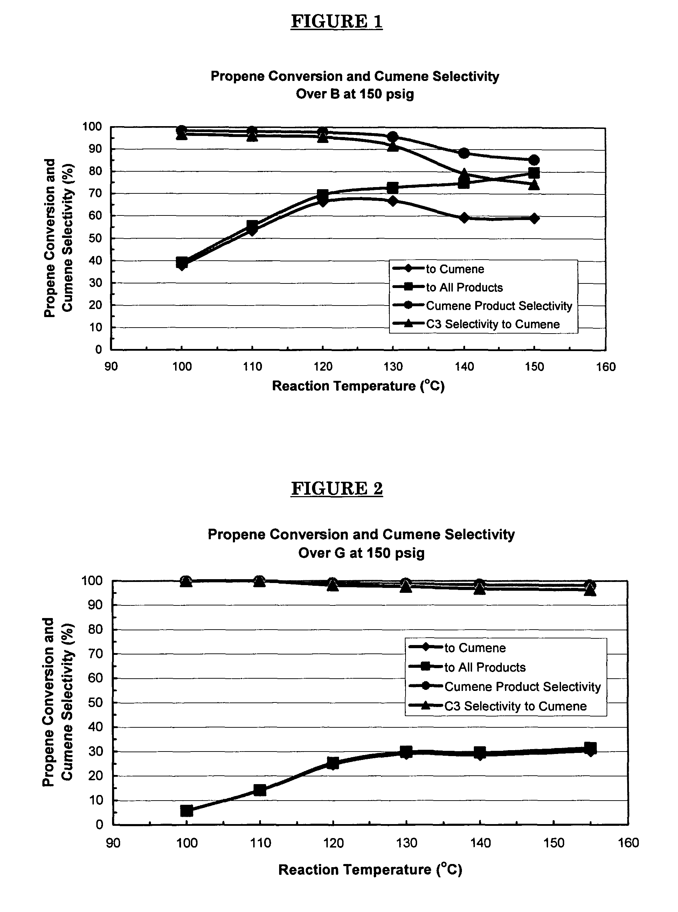

Resin **F** was also post treated using the process described in U.S. Patent No. 4,705,808 to get resin **C**. The results in Table 2 show low activity for benzene alkylation and poor stability indicated by loss in conversion of C3 on the second day. In contrast resin **B** which was prepared by the base treatment of **F** shows 4 fold higher C3 conversion. This is an unexpected result and shows that the procedures described in this invention generate catalysts with high activity for aromatic alkylation. A chlorinated, surface sulfonated resin **G** showed lower conversion than **B** but higher selectivity for end product. Figure 1 shows the catalytic activity of resin B and Figure 2 shows the activity of resin G.

Figure 1 clearly shows that that as the temperature goes beyond 130°C the propene (C3) goes more to other products rather than cumene. In contrast Figure 2 shows a different result for catalyst G.

Catalyst G appears to be very selective for cumene production even at higher temperatures indicated by the fact that C3 conversion to cumene is similar to other products. Therefore, a halogenated surface sulfonated resin provides unique properties as a catalyst for alkylation reactions. Catalysts A-E, not including C are excellent catalysts for alkylation reactions having a combination of high thermal stability, activity, selectivity, and having greatly reduced corrosive tendencies. By way of example, they operate in a temperature range from 60-200° C. Resins A-E, and not including C are used as catalysts to prepare commercially important alkylation products described herein. It is appreciated that alkylation products other than those described herein can also be prepared.

### Example 1

Polymer synthesis: Base styrene DVB co-polymers used to make catalysts of the present invention are commercially available in sulfonated forms. For example, Amberlyst™ 39 resin (commercially available from Rohm and Haas Company) was chlorinated to make resin **A** using Scheme 1. Similarly, Amberlyst 16 was used to prepare resin **F**. Treatment of resin **F** with water at 150 degrees C for ten days according to U.S. Patent No. 4,705,808 produced resin **C**. Amberlyst™ 36 resin was chlorinated to make resin **D** according to Scheme 2. For resin **E,** the substrate 4-chlorostyrene co-polymer which serves as a precursor of a catalyst of the present invention was made according to the art procedures in British Patent No. 1,393,594. The sulfonation was conducted using oleum at 110°C for 1 hour - 100g of polymer required 1000g of oleum.

### Example 2

Chlorination of polymer: The following procedure is representative for all examples and halogenations (including chlorinations). However, it is appreciated that other chlorination procedures can be used to obtain the properties of the catalyst described herein: 490 ml of wet Amberlyst ™ 36 resin is placed in a two liter glass reactor connected to a water circulation bath. 1180 g of water is added to this, and after stirring the water circulation bath is set to 35°C. The system is purged out for ten minutes with nitrogen and then chlorine is introduced into the reactor at 1.05 kg/g (15 psig). The chlorine feed rate is a function of reaction rate and can be monitored by measuring weight loss of the reaction vessel. The percent HCl in the liquid is a measure of how much chlorine is on the resin since each Cl atom on the resin produces one HCl which dissolves in the water. This serves as a tool to gauge how much chlorine is on the resin. Aliquots of solution can be removed and titrated with base to measure percentage HCl in solution. After 15 hours, the percentage HCl was found to be 2 percent. The reaction was stopped and the resin is washed with several rinses of DI water then sent for analysis. The following results were found:Cl: 11.87%, S: 16.66%

### Example 3

The following experiment illustrates that base treatment removes leachable chlorine: 75 grams of wet resin F was mixed with 220ml of aqueous 50% NaOH and heated for 4 hours at 130°C. The resin was filtered and the filtrate plus washes were weighed. A 10 g sample of the filtrate was treated with nitric acid to a pH of 2, then titrated with silver nitrate. Based on this result the amount of chlorine in 10gm of filtrate was normalized to the overall weight of filtrate plus washes. The amount of leachable chlorine in 75 g of wet resin was determined as 1.46g. The same experiment when repeated at room temperature for ten minutes gave only 0.47g of chlorine showing that the heat treatment with base removes additional chlorine from the polymer.

### Example 4

The following base treatment procedure was used to make resins **A, B, D,** and **E**. Approximately 275 grams of chlorinated resin (washed with 1500 ml of deionized water) was placed in a 3 neck round-bottomed flask equipped with a mechanical stirrer, water condensor and a thermowatch. 1192 ml of a 2N aqeous NaOH solution was added to this. The mixture was stirred and heated to reflux (103°C) for a total of 22 hrs. The solution was separated from the resin and combined with a 100 ml washing of the resin. The solution was acidified with HNO₃ and then titrated with silver nitrate for chloride determination. The resin was washed 3 times with 500 ml of deionized water then transferred to a column and washed with 3 liters of water (3 hrs) and 3 liters of 4% hydrochloric acid (3hrs) and then three liters of water(3hrs).

For resin **A** - the following was found before base treatment: 29.7% Cl, 8.2% S. After base treatment, the following results were obtained: 26.78% Cl, 8.62% S. The calculated amount of chloride recovered from the resin is 6.79g which is consistent with the reported analysis. For resin **D** - the following was found before base treatment: 11.87% Cl, 16.66% S. After base treatment, the following results were obtained: 10.04% Cl, 16.69% S. For resin **E** - the following was found before base treatment: 18.87% Cl, 13.97% S. After base treatment, the following results were obtained: 15.96% Cl, 13.95% S.

### Example 5

The following example provides one variant of a thermal stability test procedure used herein: 40 ml of resin and 28 ml of deionized water is added to a 125 ml acid digestion bomb. The bomb is sealed and placed in a vacuum oven which is then heated to 200°C. After 24 hours, the heat is turned off and the oven is cooled for 4 hours. The bomb is removed and the lid is removed in a hood. The liquid is separated from the resin and the pH is measured and the chlorine content determined by titration. The resin is washed thoroughly and its acid capacity is measured as described in example 6. The same experiment was also repeated in three smaller digestion bombs, each containing 14 ml of resin and 10 ml of water. After the thermal stability experiment the three samples were combined for analysis. Results are shown in Table 1.

### Example 6

The following procedure is a general method used to determine acid capacity of all resins. The acid capacity of resin can be determined as follows: The wet resin was placed in a beaker and dried overnight at 110°C. The beaker was placed in a desiccator, allowed to cool and weighed. The first weight observed on the balance was recorded (allowing the resin to sit exposed to the air results in re-absorption of moisture). The weight of dry solid was recorded (typically we used between 5.5 and 6.0g). After transferring to a column the resin, approximately 300 ml of deionized water was passed through the resin for half hour. Separately, a solution of sodium nitrate was prepared by dissolving 45 g in a liter of water. This flowed through the column in 2 hours and the eluate was collected in a 1000 ml volumetric flask up to the mark. A 100 ml of this sample was titrated with 0.1008 N solution of sodium hydroxide. The acid capacity was calculated as [V1*0.1008*10/W1] mmol H+ per gram of dry resin where V1 is the volume of base required for neutralization and W1 is the dry weight of resin.

### Example 7

The following procedure is used to pre-treat a catalyst before use in an alkylation reaction. A portion of the wet tan resin bead catalyst (7.2200 g) as received was put in an oven at 110°C for 8 hr. Upon removal, it was placed in a desiccator over Drierite™ desiccant, cooled and stored. The dehydration process resulted in a 52.7% weight loss. The sample was split into two portions, with one being stored in a vial in the dessicator and the other (1.8360 g) being added to 10 ml methanol at ambient temperature for swelling. After 4 hr, the methanol was decanted and the sample was dried in an oven at 60°C for nearly 3 days. After cooling in a desiccator over Drierite™ desiccant, it was shown that the methanol swelling resulted in a 6.0% wt gain.

### Example 8

The following testing conditions were employed. A 1.00 g portion of the methanol-swollen and dried catalyst, mixed with approximately 8 ml of 3 mm Pyrex beads, was loaded into the gas-phase continuous downflow 316 stainless steel tubular reactor (0.75-inch outside diameter). The reactor contained an axial thermocouple well that was positioned so that it extended from the bottom of the catalyst bed in the reactor to the top of the reactor. This provided for a sliding thermocouple to monitor and control the temperature inside of the reactor. The catalyst bed was centered in the reactor using addition 3 mm Pyrex beads above and below the catalyst bed and separated from it by layers of approximately 1 cm Pyrex wool. A double wool plug was utilized below the catalyst bed.

After loading the reactor, it was mounted in the self-contained testing system, purged with nitrogen, and then pressurized to 10.549 kg/cm (150 psig) with nitrogen at a flow rate of 130 ml/min to check for leaks. Maintaining the nitrogen gas flow at this flow rate, the reactor was slowly heated to 100°C over a period of 3 hr. Injection of benzene into the gas stream at the top of the reactor was then initiated by means of a calibrated Gilson Model 302 high pressure pump to yield a resultant gaseous benzene reactant flow rate of 70 ml/min. The liquid was vaporized in the heated inlet line to the reactor. After equilibration of this mixture, the flow of nitrogen was replaced by an equilivalent flow of a 7.75% propene/nitrogen mixture. The flow rate was adjusted to yield a reactant gas mixture of N₂/benzene/propene = 6.0/3.5/0.5.

These testing conditions corresponded to a N₂ flow rate of 120 ml/min, a propene flow rate of 10 ml/min, and a benzene gas flow rate of 70 ml/min (liquid injection rate of approximately 0.30 ml/min). These feed rates correspond to about 2.90 mmol benzene/min and about 0.41 mmol propene/min. The corresponding total gas hourly space velocity (GHSV) was 12,000 P/kg catal/hr. Testing was carried out sequentially at 100, 110, 120, 130, 140, and 150 °C. Reactant and product analyses were carried out by gas chromatography (GC) using a dimethylpolysiloxane capillary column, a 15 ΦP sample loop, and a thermal conductivity detector (TCD). The reactor exit stream was typically sampled every 20-25 min using an in-line automated heated sampling valve.

### Example 9

This is an example of a process similar to that used in U.S. Patent No. 5,863,419 using the catalysts of the present invention. A synthetic feedstock containing 0.5% thiophene and 0.45% methylthiophene and approximately 4% 1-hexene and 4% 1-heptene, balance benzene, toluene and heptane was passed over catalysts A-E and G at 200°C at 17 atm and a space velocity of 2LHSV. The resulting product analysis showed greater than 90% thiophene conversion.

In yet another variant, it is appreciated that a system for alkylation utilizing the method described herein is also provided. Use of the catalyst of the present invention provides significant benefits including a system with decreased down time due to reactor or other system element corrosion or breakdown. It is further appreciated that a manufacturing facility utilizing the system described above can operate for longer periods of time without change out of reactors or other elements effected by corrosion.

There are several exemplary down stream products that are made from the alkylation products manufactured using the present invention. These downstream products benefit from reduced or eliminated detrimental halogen contamination in the products of the alkylation reactions described herein. By way of example, cumene is used to make phenol and acetone which end up in important end products such as bis-phenol A. Bis-phenol A is then used to make a wide variety of polycarbonates. These polycarbonates are used to make compact discs and other products. Phenol can be further alkylated with octene, nonene or dodecene to make products which are used as lubricant additives, antioxidants, surfactants, fuel additives, herbicides, insecticides, fragrance and so on. Similarly, ethyl benzene is used to make styrene and cymene is used to make cresols. The alkylation of benzene with C₁₀-C₁₄ olefins are used to make linear alkylbenzenes. These are then used to make surfactant detergent intermediates. The alkylation of thiophenes with olefins is used to reduce sulfur content in gasoline as described in U.S. Patent Nos. 5,599,441, and 5,863,419. It is appreciated that in addition to these downstream products other downstream products can be produced.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

1. A method of aromatic alkylation, comprising:
adding a base treated, sulfonated, halogenated thermally stable catalyst, said catalyst comprising a styrene DVB copolymer, a styrene DVB/EVB copolymer, or a combination thereof, to reactants.

2. The method as claimed in claim 1 wherein said catalyst is surface sulfonated.

3. The method of claim 1 in which said catalyst is acid regenerated.

4. The method as claimed in claim 1 wherein the reaction takes place at a temperature in the range of 60-200°C.

5. A system for alkylation utilizing the method of claim 1.

6. A product manufactured utilizing the method of claim 1.

7. A downstream product made from a product manufactured using the method of claim 1.
